# EUROPEAN PATENT SPECIFICATION

(11) **EP 1 049 450 B1**
(45) Date of publication and mention of the grant of the patent: **13.04.2005**
(21) Application number: 98955570.1
(22) Date of filing: 09.11.1998
(51) Int. Cl.: A61K 7/42, A61K 7/06, A61K 7/48

(54) **SKIN AND HAIR DARKENING COMPOSITION**
HAUT UND HAAR TÖNENDE ZUSAMMENSETZUNG
COMPOSITION SERVANT A FONCER LA PEAU ET LES CHEVEUX

(30) Priority: 20.01.1998 GB 9801191
(43) Date of publication of application: 08.11.2000
(73) Proprietor: UNILEVER PLC, London EC4P 4BQ (GB); UNILEVER N.V., 3013 AL Rotterdam (NL)
(72) Inventor: RAGHUPATHI, Subramanian, Hindustan Lever Ltd, Mumbai 400 099 (IN); RAMAIAH, Abduri,Dept.Biochemistry,PVNR Medical.Col, Dehradadun UP 248 140 (IN); RAMAN, Govindarajan, Hindustan Lever Ltd, Bangalore 560 066 (IN); WAGH, Sushama Shripad, Hindustan Lever Ltd, Bangalore 560 066 (IN)
(74) Representative: Acham, Nicholas Clive
(86) International application number: PCT/EP1998/007218
(87) International publication number: WO 1999/037279

(56) References cited:
- EP-A- 0 293 837
- CH-A- 674 310
- FR-A- 2 608 424
- US-A- 4 866 038

## Description

The present invention relates to a cosmetic composition for darkening the skin and/or hair. The invention also relates to a method of topically applying to the skin and/or hair a skin/hair darkening composition according to the invention.

Skin tanning by UV exposure is a well known phenomenon. However, it is also well known from the literature that such exposure to UV radiation results in accelerated aging of skin and increased incidence of skin cancer. Accordingly, alternative modes of skin tanning have evolved. It is presently known in the art to use dihydroxy acetone (DHA) as a non-UV induced tanning aid. However, undesirably, the use of dihydroxy acetone for skin tanning purposes produces a rather unnatural looking sun tan. Further, the artificial tan produced by DHA does not protect against UV irradiation as would a natural tan.

Melanin is the black pigment of hair and skin and is synthesized from the amino acid tyrosine by melanosomes. Melanosomes are organelles found in melanocytes, a cell type present at dermis-epidermis junction. Tyrosine is acted upon by an enzyme, tyrosinase, which is the key step in melanogenesis.

In the melanosomes the melanin is synthesized from monomers and is transferred to the neighbouring cells called keratinocytes. The keratinocytes divide and differentiate and thus transport the melanosome to the surface of the skin. The intensity of the skin colour is directly related to the number, the size, melanin content, the rate of formation and migration/transfer of melanosomes to keratinocytes.

Several specific sequences of polyaminoacids and peptide residues are known to inhibit melanin pigmentation and have a whitening effect on the skin (JP 6345797; JP 6321757; JP 6321755; JP 5170636; US 5,126,327).

EP 293 837 A discloses a composition for preventing greying of hair and restoring greyed hair to its nature colour comprising forskolin and selected peptides.

US 4 866 038 discloses a method of normalising hypopigmentation dysfunction or darkening grey hair by topically applying a composition comprising alpha-melanotropin analogues.

The peptides described in the prior art comprise a high proportion of basic hydrophobic amino acids and have isoelectric point (pI) values greater than 5.5. These are mainly used for lightening the hyperpigmented areas associated with abnormal skin conditions.

The applicants in their co-pending British patent application 9719195.1, disclose a cosmetic composition for lightening the skin comprising from 0.1 to 10% by weight of a peptide with an isoelectric point of between 2 and 5.5. Isoelectric point (pI) is defined as the pH at which net charge on a molecule is zero. Peptides having large number of acidic amino acids like glutamic acid, aspartic acid etc. have a low pI and those having basic amino acids like lysine, arginine, histidine have a high pI.

The Applicants have found that a composition comprising peptide sequences having a isoelectric point (pI) of between 6 and 11 is capable of darkening the skin/hair.

Accordingly, the present invention relates to a cosmetic skin/hair darkening composition comprising:
(i) from 0.1 to 10%, by weight or a peptide having an isoelectric point ranging from 6 to 11;
(ii) an effective amount for tanning of an agent selected from the group consisting of dihydroxy acetone, theophylline, copper gluconate, and natural actives obtained from *Pterocarpus santalinus*; and
(iii) a cosmetically compatible carrier.

The skin/hair darkening effected by the composition of the invention is reversible and without any side effects. The composition according to the invention is active during both day and night.

The peptide is a sequence of amino acids and is of molecular weight ranging from 200 to 20,000 daltons (Da) with a pI ranging from 6.0 to 11. 0. The peptide is also optionally linked to a hydrophobic amino acid or a targeting molecule or vehicle.

The amino acid residues forming the peptide sequence can be naturally occurring or synthetic, dextro or levo form, and includes any derivative thereof. The peptide sequence must comprise a proportion of the basic amino acids such that the resulting peptide is basic in nature. The peptide sequence may be straight chain or cyclic.

The molecular weight of the peptide sequence ranges from 200 to 20,000 Da and preferably from 200 to 2000 Da.

The pI of the peptide sequence ranges from 6.0 to 11.0.

The hydrophobic amino acid can be chosen from any one of alanine, isoleucine, leucine, methionine, phenyl alanine, proline, tryptophan or valine and is preferably tryptophan. The targeting molecule is preferable a peptide and most preferably a hexapeptide preferably having the primary sequence aspargine-glutamine-proline-leucine-leucine-threonine, and is located within 27 amino acid residue from the carboxy terminal of the active peptide. Targeting vehicles such as micelles and/or reverse micelles, may also be used.

According to a preferred aspect of the invention there is provided a cosmetic skin/hair darkening composition comprising from 0.5 to 5.0% by weight of the peptide.

The invention further relates to a cosmetic method of darkening skin/hair comprising topically applying to the skin and/or hair a composition according to the invention.

The composition also comprises a skin tanning agent. This tanning agent is chosen from dihydroxy acetone, theophyllin, copper gluconate, and natural actives obtained from *Pterocarpus santalinus*.

The composition according to the invention also comprises a cosmetically compatible carrier. It may also comprise preservatives, emulsifiers, thickeners, perfume, colour, skin benefit materials such as moisturisers, emollients and antiageing compounds.

The vehicle which forms part of the cosmetic composition is one or more substances which are compatible with the polyamino acid sequence and which are also cosmetically acceptable in that they will not harm the skin/hair. The vehicles that can be used in the compositions according to the invention can include powder absorbents, binders and carriers, and liquids such as emollients, propellants, solvents, humectants and thickeners. Also simple vehicles such as alcohol, PEG, propylene glycol may also be used.

Examples of moisturisers and humectants include polyols, glycerol, cetyl alcohol, carbopol 934, ethoxylated castor oil, paraffin oils, lanolin and its derivatives. Silicone compounds such as silicone surfactants like DC3225C (Dow Corning) and/or silicone emollients, silicone oil (DC-200 Ex-Dow Corning) may also be used.

The compositions according to the invention may be prepared for topical application to the skin/hair in the form of simple solutions or conventional leave-on or wash-off products such as lotions, creams, ointments, shampoos and/or aerosol products.

All percentages referred to herein and in the appended claims are by weight of the composition unless otherwise indicated.

The invention will now be illustrated by way of Examples.

### Example 1

### In vitro demonstration of enhancement of melanin formation

The influence of a peptide sequence with pI 11.0 on the formation of melanin at pH 5 in an *in vitro* system, comparable to the pH of the melanosomal system, was analysed.

The assay conditions for the formation of melanin under in vitro conditions are as follows.

### Assay method

The control assay mixture contained 5 µmoles of DL-DOPA (Dihydroxy phenyl alanine), 20nmoles lysozyme and 3.2 units of tyrosinase in acetate buffer pH 5.0 in a test tube. A unit is defined as the amount of tyrosinase needed to convert 1 nmol DOPA in one minute. In the experimental set 11 nmoles of polylysine, a polyamino acid sequence with pI 11.0, was used in addition to the other ingredients as defined in the control. The melanin formed was washed with the acetate buffer, suspended in 1N sodium hydroxide and dissolved by heating the sample at 60°C for 5 minutes. The absorbance was measured at 400 nm.

**Table 1**

| Sample | Melanin formed A 400 |
|---|---|
| Control | 0.120 |
| In presence of polylysine | 0.168 |

The above results show that in the presence of polylysine sequence the melanin production is significantly enhanced.

The invention will now be illustrated by reference to the following example of a cosmetic cream.

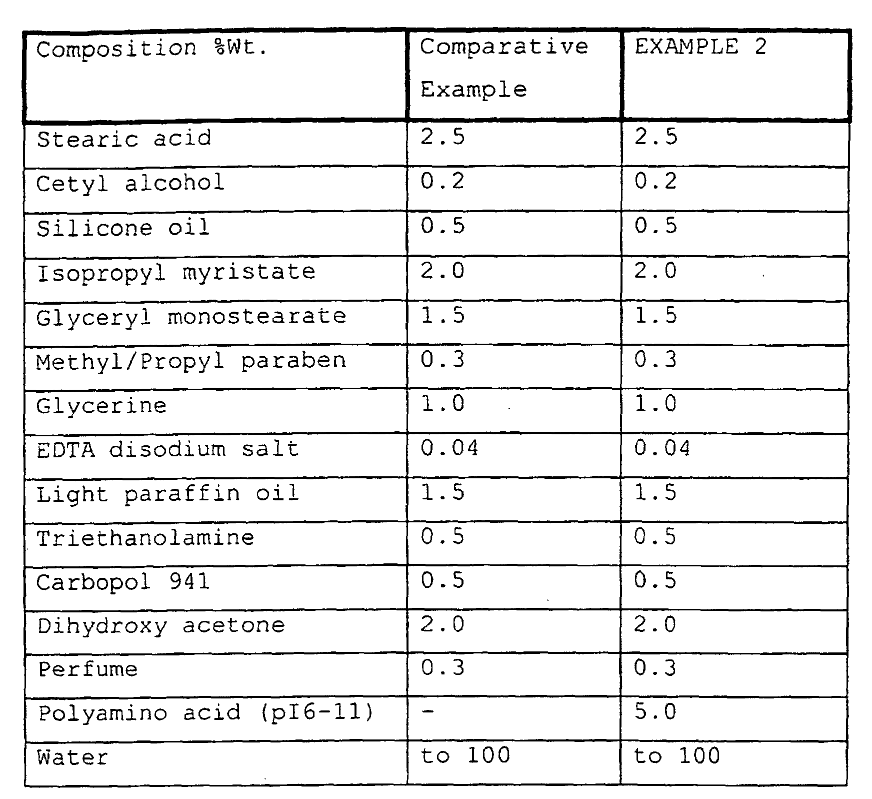

Application of the cosmetic cream described in the Comparative Example and Example 2 will show that the product described in Example 2 will be significantly superior in darkening the skin to that of the Comparative Example.

It is thus possible by way of the present invention to provide for a skin/hair darkening composition which is reversible and without any side effects. The composition is active both during day and night.

The figures in the table represent percentages of the composition by weight.

### Example 3

### In vitro demonstration of enhancement of melanin formation

The influence of the polyamino acid sequence with polyglutamate pI 2.5, polyarginine (pI 10.9) or polylysine (pI 11.0) on the formation of melanin at pH 5 in an in vitro system, comparable to the pH of the melanosomal system, was analysed. The assay conditions for the formation of melanin under *in vitro* conditions are as follows.

### Assay method:

The control assay mixture contained 5 mmoles of DL-DOPA (Dihydroxy phenyl alanine), lysozyme 20 nmoles and 0.45mg of tyrosinase in acetate buffer pH 5.0 in a test tube. In the experimental set 18 nmoles of the polyglutamate, a polyamino acid sequence with pI 2.5 or polyarginine pI 10.9 or polylysine pI 11.0 was used in addition to the other ingredients as defined in the control. The melanin formed was washed with the buffer, suspended in 1 N sodium hydroxide and dissolved by heating the sample at 60°C for 5 minutes. The absorbance was measured at 400 nm.

**Table 2**

| Sample | Melanin formed A 400 |
|---|---|
| Control | 0.120 |
| In presence of polyglutamate pI 3-4 | 0.048 |
| In presence of polylysine pI 11.0 | 0.168 |
| In presence of polyarginine pI 10.9 | 0.182 |

The above results show that in the presence of polyamino acid sequence with alkaline pI or pI > 5.0 the melanin production is significantly enhanced whereas in the presence of polyamino acid sequence with pI in the acidic range we do not get a similar enhancement in melanin production.

### Example 4

### In vivo demonstration of enhancement of melanin formation

Twelve female volunteers having even-toned skin and with no scars/visible hair on the forearms were chosen. On the volar side of the forearm 1 square cm. sites were marked using a template. A mixture of peptides of a molecular weight ranging from 14 K daltons, having a pI 11.2 at a concentration of 2% in a suitable vehicle was used. The above solution contained 0.3 µg protein/µl and 5ml of this was applied for ten days. The untreated and placebo (Vehicle) served as controls. The sites were graded by an expert, who was blinded to the treatment assignments, on zero day and on 11th day. The data is presented in table 3 shows that even under in vivo conditions peptides with a pI > 5.0 darken the skin significantly as compared to the two controls, namely the untreated and vehicle. The critical difference being 0.12

**Table 3**

| Treatment | Mean change in skin score |
|---|---|
| Control (untreated) | -0.10 ± 0.220 |
| Control (vehicle) | 0.050 ± 0.063 |
| 5% Alkaline peptide | 0.360 ± 0.074 |

| **Legends for Expert Evaluation :** | | | |
|---|---|---|---|
| SUBSTANTIALLY LIGHTENED | -1.0 | SUBSTANTIALLY DARKENED | +1.0 |
| DIFINITELY LIGHTENED | -0.75 | DIFINITELY DARKENED | +0.75 |
| MODERATELY LIGHTENED | -0.5 | MODERATELY DARKENED | +0.5 |
| SLIGHTLY LIGHTENED | -0.25 | SLIGHTLY DARKENED | +0.25 |
| NO DIFFERENCE | 0 | | |

## Claims

1. A cosmetic composition for darkening skin and/or hair comprising:
(i) from 0.1 to 10%, by weight of a peptide having an isoelectric point ranging from 6 to 11;
(ii) an effective amount for tanning of an agent selected from the group consisting of dihydroxy acetone, theophylline, copper gluconate, and natural actives obtained from *Pterocarpus santalinus*; and
(iii) a cosmetically compatible carrier.

2. The composition according to claim 1 wherein the agent is dihydroxy acetone.

3. The composition according to claim 1 or claim 2 wherein the peptide ranges in amount from 0.5 to 5.0% by weight.

4. A cosmetic composition according to any one of the preceding claims wherein the peptide has a molecular weight of from 200 to 20,000 Da.

5. A cosmetic composition according to any one of the preceding claims where the peptide is attached to either:
a) a hydrophobic amino acid chosen from alanine, isoleucine, leucine, methionine, phenylalanine, valine, proline and tryptophan; or
b) a targeting molecule or vehicle.

6. A cosmetic composition according to claim 5 wherein the hydrophobic amino acid is tryptophan.

7. A cosmetic composition according to claim 5 or 6 wherein the targeting molecule is a peptide.

8. A cosmetic composition according to claim 5, wherein the targeting molecule is a hexapeptide having the primary sequence (1):

(1)    Asx-Glm-Pro-Leu-Leu-Thr

9. A cosmetic composition according to claim 5, wherein the targeting vehicle is a micelle or reverse micelle.

10. Cosmetic method of darkening skin/hair comprising topically applying to the skin/hair a composition according to any preceding claim.

## Patentansprüche

1. Kosmetische Zusammensetzung zur Tönung der Haut und/oder des Haars, umfassend:
(i) 0,1 bis 10 Gew.-% eines Peptids mit einem isoelektrischen Punkt im Bereich von 6 bis 11;
(ii) eine wirksame Menge eines Mittels zur Bräunung, ausgewählt aus der Gruppe, bestehend aus Dihydroxyaceton, Theophyllin, Kupfergluconat und natürlichen Wirkstoffen, die aus Pterocarpus santalinus erhalten werden; und
(iii) einen kosmetisch verträglichen Träger.

2. Zusammensetzung nach Anspruch 1, wobei das Mittel Dihydroxyaceton ist.

3. Zusammensetzung nach Anspruch 1 oder Anspruch 2, wobei das Peptid in einer Menge von 0,5 bis 5,0 Gew.-% vorliegt.

4. Kosmetische Zusammensetzung nach einem der vorhergehenden Ansprüche, wobei das Peptid ein Molekulargewicht von 200 bis 20.000 Da aufweist.

5. Kosmetische Zusammensetzung nach einem der vorhergehenden Ansprüche, wobei das Peptid entweder:
a) an einer hydrophoben Aminosäure, ausgewählt aus Alanin, Isoleucin, Leucin, Methionin, Phenylalanin, Valin, Prolin und Tryptophan; oder
b) an einem Zielmolekül oder -trägerstoff angelagert ist.

6. Kosmetische Zusammensetzung nach Anspruch 5, wobei die hydrophobe Aminosäure Tryptophan ist.

7. Kosmetische Zusammensetzung nach Anspruch 5 oder 6, wobei das Zielmolekül ein Peptid ist.

8. Kosmetische Zusammensetzung nach Anspruch 5, wobei das Zielmolekül ein Hexapeptid mit der primären Sequenz ( 1 ) ist:

(1) Asx-Glm-Pro-Leu-Leu-Thr.

9. Kosmetische Zusammensetzung nach Anspruch 5, wobei der Zielträgerstoff eine Mizelle oder eine inverse Mizelle ist.

10. Kosmetisches Verfahren zur Tönung der Haut und/oder des Haars, umfassend die topische Auftragung einer Zusammensetzung nach einem der vorhergehenden Ansprüche auf die Haut oder das Haar.

## Revendications

1. Composition cosmétique pour foncer la peau et/ou les cheveux qui comprend :
(i) de 0,1 à 10 % en poids d'un peptide possédant un point isoélectrique compris dans l'intervalle de 6 à 11 ;
(ii) une quantité efficace pour bronzer d'un agent choisi dans le groupe comprenant une dihydroxy-acétone, la théophylline, le gluconate de cuivre et des substances actives naturelles obtenues à partir de Pterocarpus santalinus ; et
(iii) un support cosmétiquement compatible.

2. Composition selon la revendication 1, dans laquelle l'agent est une dihydroxy acétone.

3. composition selon la revendication 1 ou 2, dans laquelle le peptide est contenu en une quantité comprise entre 0,5 et 5,0 % en poids.

4. Composition cosmétique selon l'une quelconque des revendications précédentes, dans laquelle le peptide possède une masse moléculaire de 200 à 20.000 Da.

5. Composition cosmétique selon l'une quelconque des revendications précédentes, dans laquelle le peptide est fixé à soit :
a) un acide aminé hydrophobe choisi parmi l'alanine, l'isoleucine, la leucine, la méthionine, la phénylalanine, la valine, la proline et le tryptophane, soit
b) un véhicule ou une molécule cible.

6. Composition cosmétique selon la revendication 5, dans laquelle l'acide aminé hydrophobe est le tryptophane.

7. Composition cosmétique selon la revendication 5 ou 6, dans laquelle la molécule cible est un peptide.

8. Composition cosmétique selon la revendication 5, dans laquelle la molécule cible est un hexapeptide possédant la séquence primaire (1) :

(1) Asx-Glm-Pro-Leu-Leu-Thr

9. Composition cosmétique selon là revendication 5, dans laquelle le véhicule cible est une micelle ou une micelle réverse.

10. Procédé cosmétique pour foncer la peau/les cheveux qui consiste à appliquer par voie topique sur la peau/les cheveux une composition selon l'une quelconque des revendications précédentes.
